# EUROPÄISCHE PATENTANMELDUNG

(11) **EP 3 263 009 A2**
(43) Veröffentlichungstag der Anmeldung: **03.01.2018**
(21) Anmeldenummer: 17176331.1
(22) Anmeldetag: 16.06.2017
(51) Int. Cl.: A61B 1/00, A61B 1/06, A61B 1/12, G02B 23/24

(54) **VORRICHTUNG FÜR DIE VIDEOENDOSKOPIE**

(30) Priorität: 30.06.2016 DE 102016111960
(71) Anmelder: Karl Storz GmbH & Co. KG, 78532 Tuttlingen (DE)
(72) Erfinder: Rutschmann, Harry, 79771 Klettgau (DE); Bos, Pier, 78607 Talheim (DE); Schmal, Andreas, 78576 Emmingen-Liptingen (DE); Beutner, Dominik, 78359 Orsingen-Nenzingen (DE)
(74) Vertreter: Witte, Weller & Partner Patentanwälte mbB

(57) **Zusammenfassung**

Eine Vorrichtung für die Videoendoskopie, insbesondere für die industrielle Videoendoskopie, weist ein Geräteteil (12) auf, das ein Gehäuse (20) und eine in dem Gehäuse (20) angeordnete Elektronikbauteilanordnung (28) aufweist. Die Elektronikbauteilanordnung (28) ist innerhalb eines abgedichteten Bereichs (30) in dem Gehäuse (20) angeordnet. In dem Gehäuse (20) ist ein Kühlkörper (32) angeordnet, der mit der Elektronikbauteilanordnung (28) thermisch gekoppelt ist, um Wärme von der Elektronikbauteilanordnung (28) aufzunehmen. In dem Gehäuse (20) ist ein Lüfter (34) zum Erzeugen eines Luftstromes (42) aus Umgebungsluft angeordnet, der entlang des Kühlkörpers (32) zum Abführen der Wärme von dem Kühlkörper (32) aus dem Gehäuse (20) heraus strömt, wobei der Luftstrom (42) mit der Elektronikbauteilanordnung (28) nicht in Berührung kommt. Fig. 2

## Beschreibung

Die Erfindung betrifft eine Vorrichtung für die Videoendoskopie. Die vorliegende Erfindung betrifft insbesondere eine solche Vorrichtung für die Videoendoskopie, die in industriellen Anwendungen verwendet wird. Die Vorrichtung wird beispielsweise für die Inspektion von Maschinen, Motoren, Turbinen, Reaktoren, Bauten und dergleichen verwendet.

Eine Vorrichtung für die Videoendoskopie weist ein Endoskop auf, an das eine Kamera angeschlossen wird, oder ein Endoskop, bei dem die Kamera in das Endoskop integriert ist, sei es im distalen Bereich des Endoskopschaftes oder am proximalen Ende des Endoskopschaftes. Sowohl bei einem Endoskop mit angeschlossener Kamera als auch bei einem Endoskop mit integrierter Kamera wird das Endoskop über ein Kabel, das Signal- und Versorgungsleitungen aufweist, mit einem Geräteteil verbunden, das unter anderem der Steuerung und Versorgung der Kamera dient. Die Verbindung des Kabels mit dem Geräteteil erfolgt dabei über ein Steckerteil. Das Geräteteil weist eine Elektronikbauteilanordnung mit elektronischen Bauteilen auf, und auch das Steckerteil kann eine Elektronikbauteilanordnung mit elektronischen Bauteilen aufweisen.

Das Geräteteil kann außerdem auch eine Lichtquelle aufweisen, deren erzeugtes Licht über ein Lichtleitkabel ebenfalls mit dem Endoskop verbindbar ist.

Eine Vorrichtung für die medizinische Videoendoskopie wird von der Firma KARL STORZ GmbH & Co. KG, Tuttlingen, Deutschland, unter der Handelsbezeichnung TELE PACK X LED vertrieben. Eine Vorrichtung für die industrielle Videoendoskopie wird von der Firma KARL STORZ GmbH & Co. KG, Tuttlingen, Deutschland, unter der Handelsbezeichnung TECHNO PACK vertrieben.

Unabhängig davon, ob es sich um eine Vorrichtung für die medizinische Videoendoskopie oder eine Vorrichtung für die industrielle Videoendoskopie handelt, besteht ein Problem darin, dass die Elektronikbauteilanordnung im Gehäuse des Geräteteils während des Betriebs Wärme erzeugt. Diese Wärme ist ein ungewolltes Nebenprodukt, das die Leistungsfähigkeit der Elektronikkomponenten verringert und die Lebenszeit der Elektronikkomponenten verkürzt. Aus diesem Grund ist bei der bekannten Vorrichtung in dem Gehäuse des Geräteteils eine Kühlung vorgesehen, die einen Lüfter aufweist, der im Betrieb aus der Umgebung des Gehäuses Umgebungsluft ansaugt und einen Luftstrom erzeugt, der die Elektronikbauteilanordnung umspült und damit die Wärme von der Elektronikbauteilanordnung aufnimmt und an anderer Stelle aus dem Gehäuse des Geräteteils heraus an die Umgebung abführt.

Die Kühlung der Elektronikbauteilanordnung des Geräteteils mit Umgebungsluft, die die Elektronikbauteilanordnung umspült, erweist sich jedoch dann als problematisch, wenn die videoendoskopische Vorrichtung in rauer Umgebung betrieben wird, wie dies in industriellen Anwendungen der Fall ist. In solchen rauen Umgebungen ist die Umgebungsluft beispielsweise mit Staub, Feuchtigkeit oder salzhaltigen Stoffen versetzt. Wird die Elektronikbauteilanordnung mit der Umgebungsluft gekühlt, können die elektronischen Komponenten daher verschmutzen, korrodieren und als Folge davon Fehlfunktionen aufweisen. Das Kühlkonzept der bekannten videoendoskopischen Vorrichtungen ist daher nachteilig.

In US 8,767,060 B2 ist eine videoendoskopische Vorrichtung beschrieben, bei der zur Kühlung elektronischer Komponenten verschiedene Maßnahmen vorgesehen sind. Gemäß einer Maßnahme wird der Treiberstrom automatisch reduziert, wenn eine erhöhte Temperatur detektiert wird. Gemäß einer weiteren Maßnahme sind Wärmesenken in dem Gehäuse angeordnet, die thermisch leitende Pfade nach außen bilden.

Der Erfindung liegt die Aufgabe zugrunde, eine Vorrichtung für die Videoendoskopie anzugeben, bei der die Elektronikbauteilanordnung auch in rauen Umgebungen gekühlt werden kann, ohne durch Umgebungseinflüsse Schaden zu nehmen.

Zur Lösung dieser Aufgabe wird erfindungsgemäß eine Vorrichtung für die Videoendoskopie, insbesondere für die industrielle Videoendoskopie, bereitgestellt, mit einem Geräteteil, das ein Gehäuse und eine in dem Gehäuse angeordnete Elektronikbauteilanordnung aufweist, wobei die Elektronikbauteilanordnung innerhalb eines abgedichteten Bereichs in dem Gehäuse angeordnet ist, und wobei in dem Gehäuse ein Kühlkörper angeordnet ist, der mit der Elektronikbauteilanordnung thermisch gekoppelt ist, um Wärme von der Elektronikbauteilanordnung aufzunehmen, und wobei in dem Gehäuse ein Lüfter zum Erzeugen eines Luftstroms aus Umgebungsluft angeordnet ist, der entlang des Kühlkörpers zum Abführen der Wärme von dem Kühlkörper aus dem Gehäuse heraus strömt, wobei der Luftstrom mit der Elektronikbauteilanordnung nicht in Berührung kommt.

Bei der erfindungsgemäßen videoendoskopischen Vorrichtung ist die Elektronikbauteilanordnung gegen ihre Umgebung dicht gekapselt. Die dichte Kapselung der Elektronikbauteilanordnung ist dadurch realisiert, dass der Bereich in dem Gehäuse, in dem die Elektronikbauteilanordnung angeordnet ist, abgedichtet ist. Eine dichte Kapselung der Elektronikbauteilanordnung kann durch jedes geeignete Mittel, beispielsweise Dichtungen, realisiert sein. Unter "dicht gekapselt" ist hier zu verstehen, dass die Elektronikbauteilanordnung insbesondere gegen die Berührung mit Flüssigkeiten, Staub oder sonstige für die Elektronikbauteilanordnung schädliche Medien geschützt ist. Die Elektronikbauteilanordnung kann als Ganzes, komponentengruppenweise oder komponentenweise dicht gekapselt sein. Des Weiteren ist in dem Gehäuse ein Kühlkörper angeordnet, der mit der Elektronikbauteilanordnung thermisch gekoppelt ist. Der Kühlkörper nimmt im Betrieb Wärme von der Elektronikbauteilanordnung auf, die diese als unerwünschtes Nebenprodukt erzeugt. Die thermische Kopplung des Kühlkörpers mit der Elektronikbauteilanordnung kann dadurch realisiert sein, dass die Elektronikbauteilanordnung zumindest teilweise in Berührung mit dem Kühlkörper steht. Schließlich ist in dem Gehäuse ein Lüfter zum Erzeugen eines Luftstroms aus Umgebungsluft angeordnet, wobei der Luftstrom entlang des Kühlkörpers zum Abführen der Wärme von dem Kühlkörper aus dem Gehäuse heraus strömt. Der Luftstrom kommt dabei nicht mit der Elektronikbauteilanordnung in Berührung. Somit kann weiterhin Umgebungsluft, auch in rauen Umgebungen, zur Kühlung verwendet werden, wobei jedoch die Umgebungsluft nur mit solchen Teilen innerhalb des Gehäuses in Berührung kommt, insbesondere mit dem Kühlkörper oder mit dem Lüfter, die jedoch gegenüber Stoffen, die die Umgebungsluft mit sich führt, nicht empfindlich sind. Die erfindungsgemäße videoendoskopische Vorrichtung kann somit insbesondere in rauen Umgebungen eingesetzt werden, und eignet sich daher insbesondere für die industrielle Videoendoskopie.

Vorzugsweise ist der Kühlkörper mit der Elektronikbauteilanordnung im abgedichteten Bereich thermisch gekoppelt.

Im abgedichteten Bereich ist die im Betrieb entstehende Wärmemenge am größten, und mit der vorstehenden Maßnahme wird eine bsonders wirksame Wärmeabfuhr von der Elektronikbauteilanordnung erreicht.

In einer weiteren bevorzugten Ausgestaltung weist die videoendoskopische Vorrichtung weiterhin ein Steckerteil auf, das mit dem Geräteteil koppelbar ist.

Das Steckerteil kann dabei so ausgebildet sein, dass es in eine Buchse am Gehäuse des Geräteteils einsteckbar ist, wobei das Gehäuse des Steckerteils im eingesteckten Zustand im Wesentlichen vollständig in der Buchse aufgenommen ist.

In einer besonders bevorzugten alternativen Ausgestaltung weist jedoch das Gehäuse des Geräteteils eine Aussparung auf, wobei das Steckerteil ein Gehäuse aufweist, das in die Aussparung des Gehäuses des Geräteteils einsetzbar ist und im eingesetzten Zustand das Gehäuse des Geräteteils vervollständigt und von außen sichtbar bleibt.

Ein Vorteil dieser Ausgestaltung von Steckerteil und Geräteteilgehäuse besteht darin, dass das Steckerteil so ausgebildet werden kann, dass es in dem Inneraum seines Gehäuses weitere Komponenten enthalten kann, wie in weiteren bevorzugten Ausgestaltungen vorgesehen ist. Beispielsweise kann im Steckerteil eine Lichtquelle für die endoskopische Beleuchtung und/oder eine Laserlichtquelle, beispielsweise für Messzwecke, angeordnet sein. Es können elektrische Komponenten für die Übertragung von elektrischer Energie vom Geräteteil zu einem angeschlossenen Videoendoskop im Steckerteil enthalten sein, und/oder das Steckerteil kann elektronische Komponenten für die Datenübertragung (Steuerbefehle und Videodaten) vom Geräteteil zum Videoendoskop und vom Videoendoskop zum Geräteteil enthalten. Andererseits bleibt die gesamte Vorrichtung trotz eines größer bauenden Steckerteils insgesamt kompakt, weil das Steckerteil einen Teil des Gehäuses des Geräteteils bildet und im eingesetzten Zustand das Gehäuse des Geräteteils vervollständigt.

Eine Kontur der Aussparung im Gehäuse des Geräteteils und eine Außenkontur des Gehäuses des Steckerteils sind vorzugsweise komplementär zueinander, so dass das Steckerteil formschlüssig in die Aussparung eingreifen kann. Das Gehäuse des Steckerteils schließt dabei die Aussparung im Gehäuse des Geräteteils zumindest staubdicht ab.

Vorzugsweise erstrecken sich die Aussparung im Gehäuse des Geräteteils und das Gehäuse des Steckerteils in Richtung einer Breitenabmessung des Gehäuses des Geräteteils über zumindest die halbe Breite des Gehäuses des Geräteteils.

In dieser Ausgestaltung wird somit für die bauliche Abmessung des Steckerteils ein großer Bereich der gesamten Breite des Geräteteils genutzt, so dass in dem Gehäuse des Steckerteils ausreichend Bauraum für die zuvor angegebenen Komponenten (Lichtquelle (n), elektrische und elektronische Komponenten) zur Verfügung steht.

In einer weiteren bevorzugten Ausgestaltung weist das Gehäuse des Steckerteils eine Vielzahl von Kühlrippen auf.

Diese Ausgestaltung wird dadurch ermöglicht, dass das Gehäuse des Steckerteils im in die Aussparung des Gehäuses des Geräteteils eingesetzten Zustand von außen sichtbar bleibt, so dass dieser von außen sichtbare Teil des Gehäuses des Steckerteils vorteilhafterweise für die Abfuhr von Wärme aus dem Inneren des Steckerteils genutzt werden kann, insbesondere wenn im Steckerteil eine oder mehre're Lichtquellen integriert sind. Diese Wärmeabfuhr kann im Hinblick auf die Dimensionierung des Steckerteils sehr effizient sein.

Wie bereits zuvor erwähnt, beinhaltet das Steckerteil vorzugsweise eine Lichtquelle.

Die Lichtquelle dient beispielsweise dazu, Licht zu erzeugen, das vom Steckerteil ausgehend über das Kabel, mit dem das Videoendoskop mit der Vorrichtung verbunden ist, zum Videoendoskop geführt wird, aus dem es dann zur Ausleuchtung eines Arbeits- oder Beobachtungsraumes austritt.

Alternativ oder zusätzlich beinhaltet das Steckerteil vorzugsweise eine Laserquelle, insbesondere für ein Messsystem, insbesondere für ein Multipoint-Messsystem. Mit der erfindungsgemäßen Vorrichtung kann in dieser Ausgestaltung somit vorteilhafterweise auch eine Messlichtquelle für ein Messsystem bereitgestellt werden, die sich vorteilhafterweise im Steckerteil befindet.

Der vorstehend beschriebene Aspekt, wonach das Gehäuse des Steckerteils im mit dem Geräteteil verbundenen Zustand einen Teil des Gehäuses des Geräteteils bildet, indem das Steckerteil ein Gehäuse aufweist, das in eine Aussparung des Gehäuses des Geräteteils einsetzbar ist und im eingesetzten Zustand das Gehäuse des Geräteteils vervollständigt und von außen sichtbar bleibt, sowie die weiteren sich darauf beziehenden und vorstehend beschriebenen Aspekte werden auch ohne sämtliche Merkmale des Patentanspruchs 1 als eigenständige Erfindung angesehen.

Demnach ist hierin auch eine Vorrichtung für die Videoendoskopie, insbesondere für die industrielle Videoendoskopie, offenbart, mit einem Geräteteil, das ein Gehäuse und eine in dem Gehäuse angeordnete Elektronikbauteilanordnung aufweist, und mit einem Steckerteil, das mit dem Gehäuseteil koppelbar ist, wobei das Gehäuse des Geräteteils eine Aussparung aufweist, und wobei das Steckerteil ein Gehäuse aufweist, das in die Aussparung des Gehäuses des Geräteteils einsetzbar ist und im eingesetzten Zustand des Gehäuses des Geräteteils vervollständigt und von außen sichtbar bleibt.

In einer weiteren bevorzugten Ausgestaltung weist das Steckerteil eine weitere Elektronikbauteilanordnung auf, die in einem abgedichteten Bereich angeordnet ist, wobei das Steckerteil einen weiteren Kühlkörper aufweist, der mit der weiteren Elektronikbauteilanordnung thermisch gekoppelt ist, um Wärme von der weiteren Elektronikbauteilanordnung aufzunehmen.

Bei der erfindungsgemäßen Vorrichtung ist somit auch eine Kühlung für das Steckerteil vorgesehen. Das Steckerteil ist am Ende eines mit dem Videoendoskop oder der Kamera des Endoskops verbundenen oder verbindbaren Kabels angeordnet ist, wobei das Steckerteil mit dem Geräteteil koppelbar ist. Auch bei dem Steckerteil ist vorteilhafterweise vorgesehen, dass die Elektronikbauteilanordnung des Steckerteils gegen ihre Umgebung dicht gekapselt und mit einem weiteren Kühlkörper thermisch gekoppelt ist, um Wärme von der weiteren Elektronikbauteilanordnung aufzunehmen. Die dichte Kapselung der Elektronikbauteilanordnung des Steckerteils kann dadurch realisiert sein, dass der Stecker im Wesentlichen als Ganzes dicht gekapselt ist.

Auch bezüglich des Steckerteils ist es bevorzugt, wenn der weitere Kühlkörper mit der weiteren Elektronikbauteilanordnung im abgedichteten Bereich thermisch gekoppelt ist.

Weiterhin sind das Steckerteil und das Geräteteil bevorzugt entlang einer Achse angeordnet, wobei der Kühlkörper und der weitere Kühlkörper in Richtung der Achse aneinander anschließen, und wobei der von dem Lüfter erzeugte Luftstrom auch entlang des weiteren Kühlkörpers zum Abführen der Wärme von dem weiteren Kühlkörper strömt, ohne mit der weiteren Elektronikbauteilanordnung in Berührung zu kommen.

Diese Ausgestaltung ist besonders vorteilhaft, weil für die Erzeugung des Luftstroms zur Abführung der Wärme auch von dem weiteren Kühlkörper des Steckerteils nur ein Lüfter erforderlich ist, was den konstruktiven Aufwand der erfindungsgemäßen Vorrichtung vorteilhaft reduziert. Außerdem entsteht durch die Reihenanordnung des Kühlkörpers des Geräteteils und des weiteren Kühlkörpers des Steckerteils eine sehr kompakte Anordnung. Das Geräteteil einschließlich des Steckerteils können somit sehr kompakt bauend ausgestaltet werden, so dass sich die erfindungsgemäße Vorrichtung insbesondere als mobile, handhaltbare Vorrichtung eignet.

In einer weiteren bevorzugten Ausgestaltung weist der Kühlkörper zumindest einen Luftkanal auf, der durch das Innere des Kühlkörpers verläuft, und durch den der von dem Lüfter erzeugte Luftstrom hindurchtritt.

Durch diese Maßnahme ist der von dem Lüfter erzeugte Luftstrom noch besser von der Elektronikbauteilanordnung des Geräteteils entkoppelt, da der Luftstrom gegen die Elektronikbauteilanordnung auch durch den Kühlkörper selbst abgeschirmt ist. Außerdem kann der Luftstrom die Wärme von dem Kühlkörper besser aufnehmen, da die gesamte Luft mit dem Kühlkörper in Kontakt ist.

Die gleiche Maßnahme ist auch in Bezug auf den weiteren Kühlkörper des Steckerteils vorteilhaft, der entsprechend zumindest einen weiteren Luftkanal aufweist, der durch das Innere des zweiten Kühlkörpers verläuft, und durch den der von dem Lüfter erzeugte Luftstrom hindurchtritt.

Dabei ist es weiterhin bevorzugt, wenn der zumindest eine Luftkanal im Kühlkörper des Geräteteils mit dem zumindest einen weiteren Luftkanal im weiteren Kühlkörper des Steckerteils kommuniziert.

Auf diese Weise kann der von dem Lüfter erzeugte Luftstrom durch den Kühlkörper und den weiteren Kühlkörper entlang eines gemeinsamen Luftpfades geführt werden.

Der Lüfter ist vorzugsweise an einem gehäuseseitigen Ende des Geräteteils angeordnet.

In dieser Anordnung kann der Lüfter, beispielsweise durch ein im Gehäuse des Geräteteils vorhandenes Lüftungsgitter, Umgebungsluft ansaugen und durch das Geräteteil entlang des Kühlkörpers und gemäß einer der oben genannten Ausgestaltungen auch entlang des weiteren Kühlkörpers des Steckerteils strömen lassen.

Dabei ist es weiterhin bevorzugt, wenn das Steckerteil an einem gehäuseseitigen Ende des Geräteteils angeordnet ist, das vom Lüfter abgewandt ist.

Hierdurch wird in kompakter Bauweise der von dem Lüfter erzeugte Luftstrom von einer ersten Gehäuseseite bis zur gegenüberliegenden Gehäuseseite, an der das Steckerteil angeordnet ist, geführt, ohne dass mehrere Lüfter oder andere aufwändige Maßnahmen zur Luftstromführung erforderlich sind.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: Eine videoendoskopische Vorrichtung mit einem Geräteteil und einem Endoskop;
- Fig. 2: einen Schnitt durch das Geräteteil in Fig. 1 entlang der in Fig. 1 gezeigten Schnittlinie II, wobei ein Steckerteil der videoendoskopischen Vorrichtung mit dem Geräteteil gekoppelt ist;
- Fig. 3: eine videoendoskopische Vorrichtung mit einem Geräteteil und einem Steckerteil gemäß einem weiteren Ausführungsbeispiel, wobei das Steckerteil vom Geräteteil abgenommen ist;
- Fig. 4: die videoendoskopische Vorrichtung gemäß Fig. 3, wobei das Steckerteil mit dem Geräteteil gekoppelt ist;
- Fig. 5: eine Rückansicht der videoendoskopischen Vorrichtung in Fig. 4; und
- Fig. 6: eine teilweise aufgebrochene Darstellung der videoendoskopischen Vorrichtung in Fig. 3 bis 5.

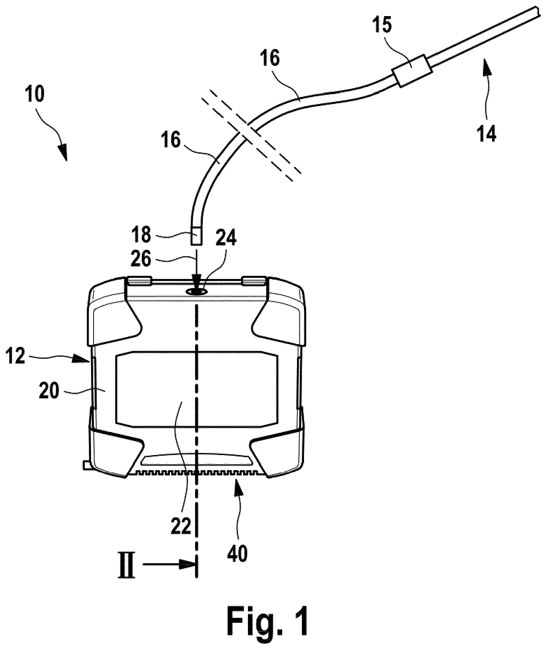

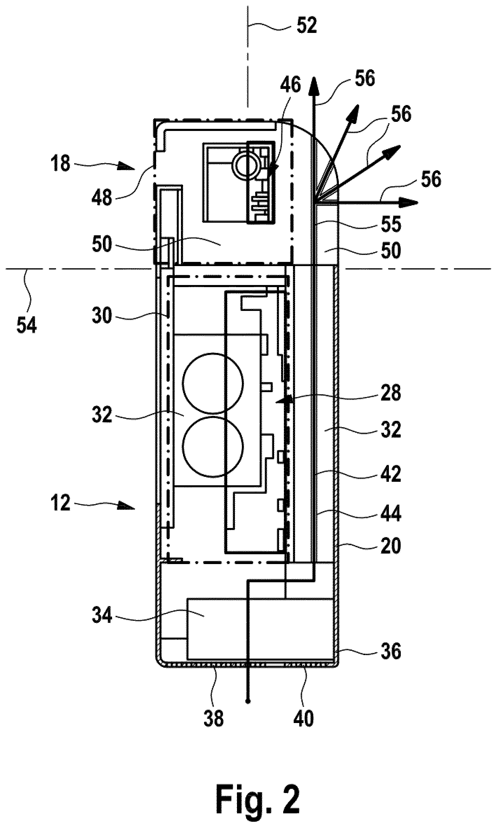

In Fig. 1 ist eine mit dem allgemeinen Bezugszeichen 10 versehene Vorrichtung für die Videoendoskopie gezeigt. Weitere Einzelheiten der Vorrichtung 10 sind in Fig. 2 gezeigt.

Die videoendoskopische Vorrichtung 10 wird insbesondere in industriellen Anwendungen zur visuellen Inspektion verwendet. Die videoendoskopische Vorrichtung 10 ist insbesondere dazu ausgelegt, in rauen Umgebungsbedingungen verwendet zu werden.

Die videoendoskopische Vorrichtung 10 weist ein Geräteteil 12 auf. Außerdem ist in Fig. 1 ein Endoskop 14 gezeigt, das als Videoendoskop mit integrierter Kamera oder als Endoskop mit angeschlossener Kamera, beispielsweise einer Kamera 15, ausgebildet ist. Das Endoskop 14 ist mit einem Kabel 16 verbunden oder verbindbar, das am proximalen Ende ein Steckerteil 18 aufweist.

Das Geräteteil 12 weist ein Gehäuse 20 auf. In dem Gehäuse 20 ist ein Anzeige- bzw. Bildschirm 22 angeordnet.

Am Gehäuse 20 ist weiterhin eine Steckerbuchse 24 angeordnet, in die das Steckerteil 18 einsteckbar ist, so dass das Steckerteil 18 mit dem Geräteteil 12 gekoppelt werden kann. In Fig. 1 ist dies mit einem Pfeil 26 veranschaulicht.

Gemäß Fig. 2 ist in dem Gehäuse 20 des Geräteteils 12 eine Elektronikbauteilanordnung 28 angeordnet, die ein oder mehrere elektronische Bauteile aufweist. Da das Gerät 12 vor allem die Funktion als Kamerasteuereinheit für die Endoskopkamera hat, ist die Elektronikbauteilanordnung beispielsweise Teil der Steuerelektronik für die Endoskopkamera.

Im Betrieb der videoendoskopischen Vorrichtung 10 erzeugt die Elektronikbauteilanordnung 28 Wärme als unerwünschtes Nebenprodukt.

Die Elektronikbauteilanordnung 28 ist in dem Gehäuse 20 des Geräteteils 12 gegen ihre Umgebung dicht gekapselt, wie mit einer strichpunktierten Umrandungslinie 30 veranschaulicht ist, die einen abgedichteten Bereich innerhalb des Gehäuses 20 darstellt. Die dichte Kapselung der Elektronikbauteilanordnung 28 kann beispielsweise durch ein oder mehrere Dichtungselemente entlang der Umrandungslinie 30 realisiert sein. Die dichte Kapselung der Elektronikbauteilanordnung 28 bewirkt, dass die Elektronikbauteilanordnung 28 nicht mit Staub, Flüssigkeiten, Sprühnebeln und dergleichen, die von der Umgebungsluft in das Innere des Gehäuses 20 mitgeschleppt werden können, in Berührung kommt.

In dem Gehäuse 20 des Geräteteils 12 ist ein Kühlkörper 32 angeordnet, der mit der Elektronikbauteilanordnung 28 thermisch gekoppelt ist, um Wärme von der Elektronikbauteilanordnung 28 aufzunehmen. Die thermische Kopplung zwischen dem Kühlkörper 32 und der Elektronikbauteilanordnung 28 kann durch Wärmestrahlung, Wärmekonvektion oder durch thermisch leitende Verbindung oder durch mehrere dieser Kopplungsarten realisiert sein. Insbesondere ist der Kühlkörper 32 mit der Elektronikbauteilanordnung 28 im abgedichteten Bereich 30 thermisch gekoppelt.

In dem Gehäuse 20 des Geräteteils 12 ist weiterhin ein Lüfter 34 angeordnet, und zwar an einem gehäuseseitigen Ende 36 des Gehäuses 20. An dem Ende 36 des Gehäuses 20 sind Lüftungsöffnungen 38 vorhanden, beispielsweise in Form eines Lüftungsgitters 40 (siehe auch Fig. 1). Der Lüfter 34 saugt im Betrieb über die Lüftungsöffnungen 38 Umgebungsluft an. Der Lüfter 34 erzeugt aus der angesaugten Umgebungsluft einen Luftstrom, der in Fig. 2 mit einer Linie 42 angedeutet ist.

Der Luftstrom 42 strömt dabei durch zumindest einen Luftkanal 44, der durch das Innere des Kühlkörpers 32 verläuft.

Der Luftstrom 42 kommt aufgrund der dichten Kapselung der Elektronikbauteilanordnung 28 mit der Elektronikbauteilanordnung 28 nicht in Berührung, da der Luftstrom 42 nicht in den abgedichteten Bereich 30 eindringen kann.

Fig. 2 zeigt das Steckerteil 18 im an das Geräteteil 12 gekoppelten Zustand. Das Steckerteil 18 weist ebenfalls eine Elektronikbauteilanordnung 46 auf, die eine oder mehrere elektronische Bauteile aufweist. Die Elektronikbauteilanordnung 46 des Steckerteils 18 ist gegen ihre Umgebung dicht gekapselt, wie mit einer strichpunktierten Umrandungslinie 48 veranschaulicht ist, die den abgedichteten Bereich des Steckerteils 18 darstellt. Die dichte Kapselung des Steckerteils 18 kann dadurch realisiert sei, dass das Steckerteil 18 insgesamt nach außen abgedichtet ist.

Das Steckerteil 18 weist einen Kühlkörper 50 auf, der mit der Elektronikbauteilanordnung 46 thermisch gekoppelt ist, um Wärme von der Elektronikbauteilanordnung 46 aufzunehmen. Die thermische Kopplung zwischen dem Kühlkörper 50 und der Elektronikbauteilanordnung 46 kann wie oben für den Kühlkörper 32 und die Elektronikbauteilanordnung 28 des Geräteteils 12 beschrieben, durch Wärmeleitung, Wärmekonvektion und/oder Wärmestrahlung realisiert sein. Die thermische Kopplung erfolgt auch hier wieder vor allem im abgedichten Bereich 48 des Steckerteils 18.

Wie aus Fig. 2 hervorgeht, sind das Steckerteil 18 und das Geräteteil 12 entlang einer Achse 52 angeordnet, wobei der Kühlkörper 50 des Steckerteils 18 und der Kühlkörper 32 des Geräteteils 12 in Richtung der Achse 52 aneinander anschließen, jedoch voneinander getrennt sind, wie in Fig. 2 mit einer weiteren Achse 54 angedeutet ist. Die Achse 54 ist die Trennlinie zwischen dem Steckerteil 18 und dem Geräteteil 12.

Der von dem Lüfter 34 erzeugte Luftstrom strömt auch entlang des Kühlkörpers 50 zum Abführen der Wärme von dem Kühlkörper 50, ohne mit der Elektronikbauteilanordnung 46 in Berührung zu kommen, da diese gegen ihre Umgebung dicht gekapselt ist. Der Kühlkörper 50 weist wie der Kühlkörper 32 zumindest einen Luftkanal 55 auf, der durch das Innere des Kühlkörpers 50 verläuft, und durch den der von dem Lüfter 34 erzeugte Luftstrom hindurchtritt. Aus dem Kühlkörper 50 tritt der Luftstrom gemäß Strömungspfeilen 56 in die Umgebung aus. Dadurch, dass das Steckerteil 18 entlang der Achse 52 an das Geräteteil 12 anschließt, und der Lüfter 34 an dem dem Steckerteil 18 abgewandten Ende 36 des Gehäuses 20 des Geräteteils 12 angeordnet ist, entsteht eine kompakte Bauweise der videoendoskopischen Vorrichtung 10, bei der nur der eine Lüfter 34 benötigt wird, um zusätzlich auch das Steckerteil 18 zu kühlen.

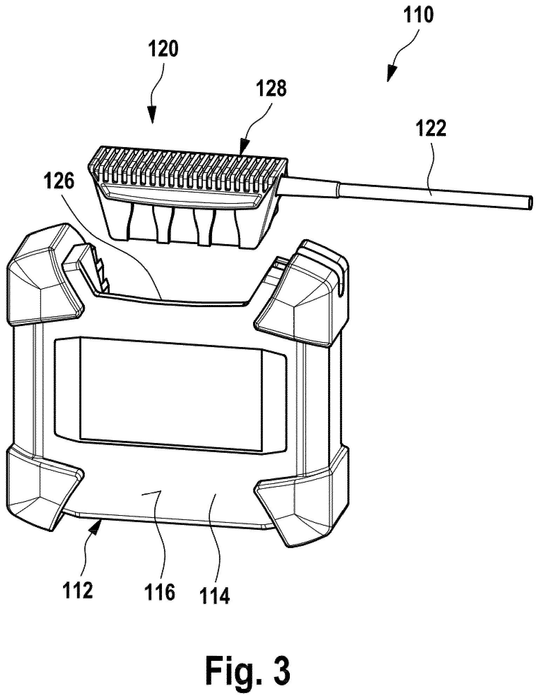

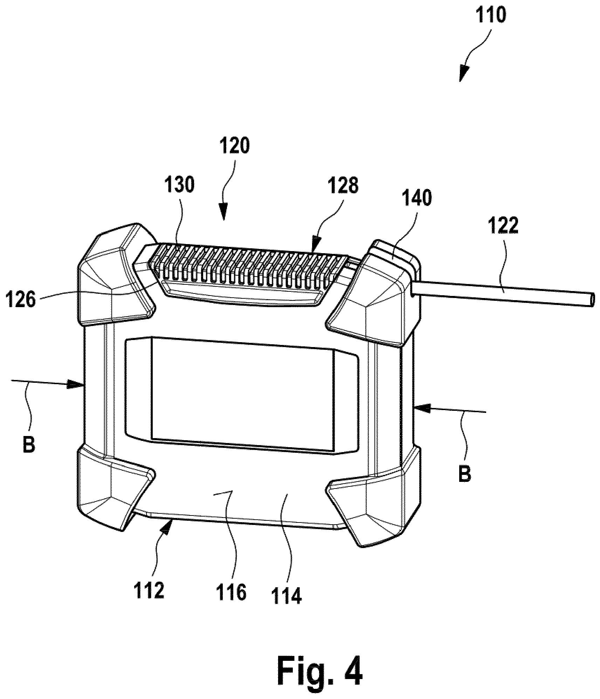

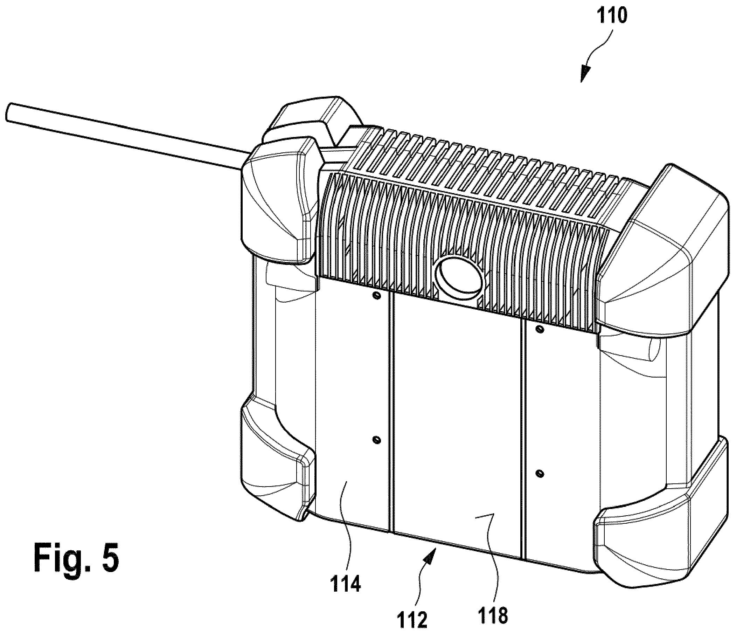

In Fig. 3 bis 6 ist ein weiteres Ausführungsbeispiel einer mit dem allgemeinen Bezugszeichen 110 versehenen Vorrichtung für die Videoendoskopie gezeigt. Die Vorrichtung 110 weist ein Geräteteil 112 auf, das ein Gehäuse 114 aufweist. Das Gehäuse 114 weist eine Vorderseite 116 auf, die in Fig. 3 und 4 zu sehen ist, und eine Rückseite 118, die in Fig. 5 zu sehen ist.

Die Vorrichtung 110 weist ein Steckerteil 120 auf. Das Steckerteil 120 ist mit einem Kabel 122 verbunden oder verbindbar, wobei das Kabel 122 mit einem Endoskop oder Videoendoskop (in Fig. 3 bis 6 nicht dargestellt), beispielsweise mit dem Endoskop 14 in Fig. 1, verbindbar ist.

Das Steckerteil 120 ist mit dem Geräteteil 112 koppelbar, insbesondere zusammensteckbar. Fig. 3 zeigt das Steckerteil 120 im vom Geräteteil 112 abgenommenen Zustand, während Fig. 4 und Fig. 5 sowie Fig. 6 das Steckerteil 120 im mit dem Geräteteil 112 verbundenen Zustand zeigen.

Das Gehäuse 114 des Geräteteils 112 weist eine Aussparung 126 auf, in die das Steckerteil 120 einsetzbar, insbesondere formschlüssig einsetzbar ist. Das Steckerteil 120 weist dazu ein Gehäuse 128 auf, das in die Aussparung 126 des Gehäuses 114 des Geräteteils 112 einsetzbar ist und damit das Gehäuse 114 des Geräteteils 112 vervollständigt und schließt. Im in die Aussparung 126 des Gehäuses 114 eingesetzten Zustand des Steckerteils 120 bleibt ein Teil des Gehäuses 128 des Steckerteils 120 von außen sichtbar. Der sichtbare Teil des Gehäuses 128 des Steckerteils 120 vervollständigt dabei das Gehäuse 114 des Geräteteils 112, im gezeigten Ausführungsbeispiel vervollständigt das Gehäuse 128 des Steckerteils 120 das übrige Gehäuse 114 des Geräteteils 112 komplementär. Mit anderen Worten bildet das Gehäuse 128 des Steckerteils 120 im eingesetzten Zustand des Steckerteils 120 einen Teil des Gehäuses 114 des Geräteteils 112. Das Gehäuse 128 schließt im eingesetzten Zustand die Aussparung 126 zumindest staubdicht, vorzugsweise auch luft- und flüssigkeitsdicht, ab.

Das Gehäuse 128 des Steckerteils 120 erstreckt sich in Richtung einer Breitenabmessung des Gehäuses 114 des Geräteteils 112, die in Fig. 4 mit zwei Pfeilen B veranschaulicht ist, zumindest über die Hälfte dieser Breitenabmessung (vgl. hierzu auch die Rückansicht in Fig. 5).

Der im eingesteckten Zustand des Steckerteils 120 sichtbar bleibende Teil des Gehäuses 128 des Steckerteils 120 weist eine Vielzahl von Kühlrippen 130 auf. Diese Kühlrippen 130 dienen der Wärmeabfuhr aus dem Inneren des Steckerteils 120.

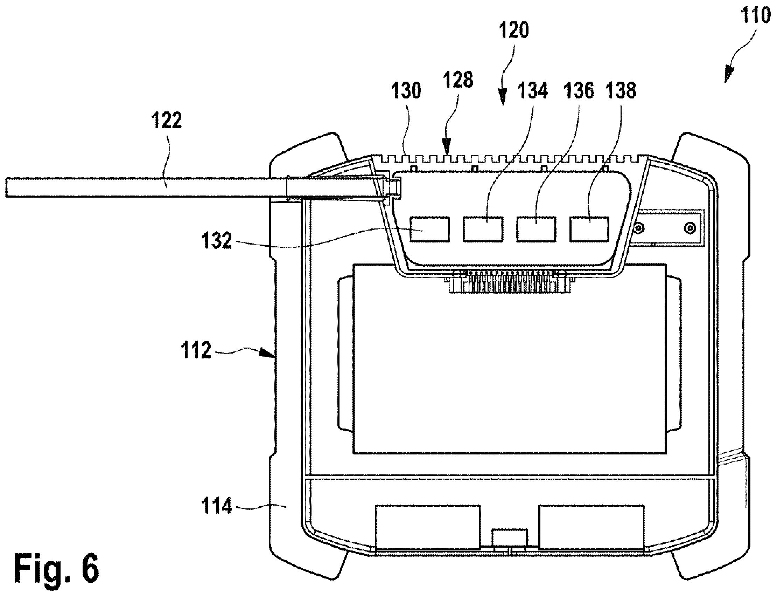

Gemäß Fig. 6 sind im Inneren des Steckerteils 120 verschiedene Komponenten angeordnet, die in Fig. 6 schematisch dargestellt sind.

Zu diesen Komponenten können elektrische Komponenten 132 gehören, die beispielsweise der Übertragung von elektrischer Energie von dem Geräteteil 112 zum Videoendoskop (beispielsweise Videoendoskop 14 in Fig. 1) dienen. Des Weiteren können in dem Steckerteil 120 elektronische Komponenten 134 angeordbet sein, die für eine bidirektionale Datenübertragung, beispielsweise für eine Übertragung von Steuerbefehlen vom Geräteteil 112 zum Videoendoskop und/oder zur Übertragung von Videodaten vom Videoendoskop zum Geräteteil 112 dienen können. Die Übertragung elektrischer Energie bzw. von Daten erfolgt dabei über das Kabel 122.

In dem Steckerteil 120 ist des Weiteren vorzugsweise eine Lichtquelle 136, vorzugsweise mit hoher Lichtausbeute, angeordnet, deren erzeugtes Licht ebenfalls über das Kabel 122 zum Videoendoskop zur Ausleuchtung eines Arbeits- bzw. Beobachtungsraums übertragen wird.

Des Weiteren kann in dem Steckerteil 120 eine Laserquelle 138 angeordnet sein, die Laserlicht für ein Messsystem, insbesondere ein Multipoint-Messsystem bereitstellt.

Die Unterbringung der vorstehend genannten Komponenten in dem Steckerteil 120 wird durch die erfindungsgemäß vorgesehene Integration des Gehäuses 128 in das Gehäuse 114 des Geräteteils 112 ermöglicht, ohne dass dabei die Vorrichtung 110 als Ganzes größer bauen müsste.

Über die Kühlrippen 130 kann vorteilhafterweise Wärme nicht nur vom Innenraum des Steckerteils 120, sondern auch vom Innenraum des Geräteteils 112 abgeführt werden. Das Geräteteil 112 kann hinsichtlich eines Kühlkonzeptes im Wesentlichen dem Geräteteil 12 der Vorrichtung 10 entsprechen, wie mit Bezug auf Fig. 1 und 2 beschrieben wurde.

Im Gehäuse 114 des Geräteteils 112 ist des Weiteren im Bereich einer Ecke eine Nut 140 ausgespart, in die das Kabel 122 eingelegt ist, wenn das Steckerteil 120 mit dem Geräteteil 112 verbunden ist.

Im Übrigen versteht es sich, dass die Ausgestaltung des Steckerteils 120 und des Gehäuses 114 mit der Aussparung 126 auch bei der Vorrichtung 10 in Fig. 1 und 2 realisiert werden kann, wobei das mit Bezug auf Fig. 1 beschriebene Kühlkonzept und das Konzept der Abdichtung von Elektronikbauteilanordnungen bestehen bleiben kann.

## Patentansprüche

1. Vorrichtung für die Videoendoskopie, insbesondere für die industrielle Videoendoskopie, mit einem Geräteteil (12), das ein Gehäuse (20) und eine in dem Gehäuse (20) angeordnete Elektronikbauteilanordnung (28) aufweist, wobei die Elektronikbauteilanordnung (28) innerhalb eines abgedichteten Bereichs (30) in dem Gehäuse (20) angeordnet ist, und wobei in dem Gehäuse (20) ein Kühlkörper (32) angeordnet ist, der mit der Elektronikbauteilanordnung (28) thermisch gekoppelt ist, um Wärme von der Elektronikbauteilanordnung (28) aufzunehmen, und wobei in dem Gehäuse (20) ein Lüfter (34) zum Erzeugen eines Luftstromes (42) aus Umgebungsluft angeordnet ist, der entlang des Kühlkörpers (32) zum Abführen der Wärme von dem Kühlkörper (32) aus dem Gehäuse (20) heraus strömt, wobei der Luftstrom (42) mit der Elektronikbauteilanordnung (28) nicht in Berührung kommt.

2. Vorrichtung nach Anspruch 1, wobei der Kühlkörper (32) mit der Elektronikbauteilanordnung (28) im abgedichteten Bereich (30) thermisch gekoppelt ist.

3. Vorrichtung nach Anspruch 1 oder 2, weiterhin mit einem Steckerteil (18), das mit dem Geräteteil (12) koppelbar ist.

4. Vorrichtung nach Anspruch 3, wobei das Gehäuse (114) des Geräteteils (112) eine Aussparung (126) aufweist, und wobei das Steckerteil (120) ein Gehäuse (128) aufweist, das in die Aussparung (126) des Gehäuses (114) des Geräteteils (112) einsetzbar ist, und wobei zumindest ein Teil des Gehäuses (128) des Steckerteils (120) im in die Aussparung (126) eingesetzten Zustand das Gehäuse (114) des Geräteteils (112) vervollständigt und von außen sichtbar bleibt.

5. Vorrichtung nach Anspruch 4, wobei sich die Aussparung (126) und das Gehäuse (128) des Steckerteils (120) in Richtung einer Breitenabmessung (B) des Gehäuses (114) des Geräteteils (112) über zumindest die halbe Breite des Gehäuses (114) des Geräteteils (112) erstrecken.

6. Vorrichtung nach einem der Ansprüche 3 bis 5, wobei das Gehäuse (128) des Steckerteils (120) eine Vielzahl von Kühlrippen (130) aufweist.

7. Vorrichtung nach einem der Ansprüche 3 bis 6, wobei das Steckerteil (120) eine Lichtquelle (136) beinhaltet.

8. Vorrichtung nach einem der Ansprüche 3 bis 7. wobei das Steckerteil (120) eine Laserquelle (138), insbesondere für ein Messsystem, beinhaltet.

9. Vorrichtung nach einem der Ansprüche 3 bis 8, wobei das Steckerteil (18)eine weitere Elektronikbauteilanordnung (46) aufweist, die in einem abgedichteten Bereich (48) angeordnet ist, wobei das Steckerteil (18) einen weiteren Kühlkörper (50) aufweist, der mit der weiteren Elektronikbauteilanordnung (46) thermisch gekoppelt ist, um Wärme von der weiteren Elektronikbauteilanordnung (46) aufzunehmen.

10. Vorrichtung nach Anspruch 9, wobei der weitere Kühlkörper (50) mit der weiteren Elektronikbauteilanordnung (46) im abgedichteten Bereich (48) thermisch gekoppelt ist.

11. Vorrichtung nach Anspruch 8 oder 9, wobei das Steckerteil (18) und das Geräteteil (12) entlang einer Achse (52) angeordnet sind, wobei der Kühlkörper (32) und der weitere Kühlkörper (50) in Richtung der Achse (50) aneinander anschließen, und wobei der von dem Lüfter (34) erzeugte Luftstrom (42) auch entlang des weiteren Kühlkörpers (50) zum Abführen der Wärme von dem weiteren Kühlkörper (50) strömt, ohne mit der weiteren Elektronikbauteilanordnung (46) in Berührung zu kommen.

12. Vorrichtung nach einem der Ansprüche 1 bis 11, wobei der Kühlkörper (32) zumindest einen Luftkanal (44) aufweist, der durch das Innere des Kühlkörpers (32) verläuft, und durch den der von dem Lüfter (34) erzeugte Luftstrom (42) hindurchtritt.

13. Vorrichtung nach einem der Ansprüche 9 bis 12, sofern auf Anspruch 9 rückbezogen, wobei der weitere Kühlkörper (50) zumindest einen weiteren Luftkanal (55) aufweist, der durch das Innere des weiteren Kühlkörpers (50) verläuft, und durch den der von dem Lüfter (34) erzeugte Luftstrom (42) hindurchtritt.

14. Vorrichtung nach Anspruch 12 und 13, wobei der zumindest eine Luftkanal (44) mit dem zumindest einen weiteren Luftkanal (55) kommuniziert.

15. Vorrichtung nach einem der Ansprüche 1 bis 14 wobei der Lüfter (34) an einem gehäuseseitigen Ende (36) des Geräteteils (12) angeordnet ist.

16. Vorrichtung nach einem der Ansprüche 3 bis 15, sofern auf Anspruch 3 rückbezogen, wobei das Steckerteil (18) an einem gehäuseseitigen Ende des Geräteteils (12) angeordnet ist, das vom Lüfter (34) abgewandt ist.
